# EUROPEAN PATENT SPECIFICATION

(11) **EP 3 846 686 B1**
(45) Date of publication and mention of the grant of the patent: **27.05.2026**
(21) Application number: 19790057.4
(22) Date of filing: 30.08.2019
(51) Int. Cl.: A61B 5/291, A61B 5/377

(54) **APPARATUS FOR ASSESSING EMOTION OF INFANTS AND YOUNG CHILDREN**
VORRICHTUNG ZUR BEURTEILUNG DER EMOTIONEN VON SÄUGLINGEN UND KLEINKINDERN
APPAREIL POUR ÉVALUER L'ÉMOTION DES NOURRISSONS ET DES JEUNES ENFANTS

(30) Priority: 04.09.2018 US 201862726643 P
(43) Date of publication of application: 14.07.2021
(73) Proprietor: Kenvue Brands LLC, Summit, NJ 07901 (US)
(72) Inventor: COUBART, Aurelie, 27100 Val De Reuil (FR); GIL-LÓPEZ, Cristina, 27100 Val De Reuil (FR); ZUNINO, Helene, 27100 Val De Reuil (FR)
(74) Representative: Carpmaels & Ransford LLP
(86) International application number: PCT/IB2019/057327
(87) International publication number: WO 2020/049430

(56) References cited:
- WO-A1-2004/000115
- US-A1- 2013 060 158
- US-A1- 2014 128 763
- US-A1- 2015 018 705
- US-A1- 2017 151 408
- US-B1- 8 805 489

## Description

### TECHNICAL FIELD

The present disclosure relates to portable electroencephalography (EEG) headsets for use in research of cognitive and emotional development of infants and young children.

### DESCRIPTION OF RELATED ART

Emotion development is an important prerequisite for infants' cognitive optimal growing. This maturational process is based on formation and synchronization of brain networks that ultimately will characterize the cognitive functioning. Electroencephalographic (EEG) is a suitable technique to explore cognitive and emotional correlates of brain activity. Nonetheless, the use of this technique in infants is limited by several practical and neurodevelopment factors in comparison to EEG research in adults. The age factor and EEG pattern stability may play an important role to ensure the validation of infants' data collection and functional interpretation of underlying brain processes. There are technical and procedural obstacles when conducting EEG research in infants' emotion development.

Conventional electroencephalography (EEG) systems use scalp level electrodes typically attached to elastic caps or bands to monitor neurological activity. Conductive gels and pastes are applied before placement of the scalp electrodes to improve sensitivity. However, application of conductive gels and pastes to infants and young children is often inconvenient, time consuming and undesirable.

Efforts have been made in the development of more portable, efficient, and effective EEG data collection mechanisms.

U.S. Patents Nos. 9,336,535 and 8,655,428 to The Nielsen Company disclose systems for gathering neuro-response data that comprise a headset to gather first neuro-response data and second neuro-response data from a user when the user is exposed to stimulus material, wherein the headset includes a band, a first sensor and a second sensor each extending from the band, wherein the first sensor and the second sensor are rotatably positioned relative to the band, a processor. Elastic caps or bands can be uncomfortable for prolonged use.

U.S. Reissue Patent No. Re. 34,015 to the Children's Medical Center Corporation discloses an apparatus for generating a topographic display of information on brain electrical activity based on responses of electrical-activity transducers placed on the skull.

U.S. Patent No. 9,770,184 to Bittium Bioisignals OY discloses attachment of an electrode configuration to hairless areas of a patient's head to produce high-quality measuring information.

U.S. Patent No. 9,370,313 and EP3087918A1 to Bio-signal Group Corp. disclose a device for performing electroencephalography on a patient that comprises a sagittal portion comprising: i) a forehead anchor comprising two arms, a first forehead anchor arm and an opposing second forehead anchor arm; ii) a neck anchor comprising two arms, a first neck anchor arm and an opposing second neck anchor arm; iii) a midsection between the forehead anchor and the neck anchor; and iv) a plurality of imbedded electrodes within the forehead anchor, and the midsection. The device further comprises: a coronal portion comprising a plurality of imbedded electrodes; and electrical connectors for electrically connecting the imbedded electrodes in the sagittal portion and the coronal portion to an electroencephalograph.

French Patent No. FR2975276B1 and WO2012156643A1 to University de Picardie Jules Verne and Centre Hospitalier Universitaire d'Amiens discloses a device for measuring the brain activity signals of an individual that comprises a supporting structure (2) intended for carrying sensors, characterized in that the supporting structure (2) forms a framework which has linking elements (5) and is hollowed out between said linking elements, wherein the linking elements (5) form at least one central chain (50) and lateral chains (51), wherein the lateral chains (51) are arranged on either side of the central chain and have gaps between them and are connected via connecting elements (8), and wherein the lateral chains (51) are articulated relative to the central chain (50) and preferably exhibit an articulation about at least two axes of pivoting.

WO2013124366A1 and GB2499595A to James I.E. Roche disclose an electroencephalogram electrode system for use in neonatal brain monitoring that comprises a deformable substrate shaped and dimensioned to conform to a head of an infant, the substrate preferably defining four lobes, the system further comprising an array of electrodes provided about the lobes of the substrate and capable of making electrical contact with the head when the substrate is positioned thereon.

U.S. Patent No. 7,415,305 and US20060074336A1 to The Trustees of Columbia University in the City of New York disclose methods and systems for monitoring and evaluating brain electrical activity. The reference discloses that the system comprises an EEG device for obtaining EEG information, the device comprising a plurality of electrodes placed to measure a subject's brain electrical activity; and a processing device for processing the EEG information to obtain information including local synchrony information, wherein the processing device is configured to execute a computer algorithm to reduce residual volume conduction artifact in the obtained local synchrony information. "Local synchrony information" is defined as information that provides a quantitative measure of local synchrony, in which adjacent electrodes (or other means for obtaining electrical activity measurement at a site) used in calculating the quantitative measure are spaced no more than 3 centimeters apart. **In** some embodiments, local coherence is used as a measure of local synchrony.

WO2007006976A3 and FR2888487A1 to University de Picardie Jules Verne and Centre Hospitalier Universitaire d' Amiens discloses a head cap for measuring the brain activity of an individual, comprising means for measuring brain activity (3) and a surrounding element (2) which can be positioned on the person's skull. The reference discloses that the surrounding element (2) is made fully or partially from a material in the form of a deformable shape memory paste which can be molded to the individual's skull.

CN103989473B to Ying Xue discloses a method to position correctly the electrodes on human's head.

WO2008058343A1 to the University of Queensland discloses a method for detecting a seizure in a newborn or a young child.

U.S. Published Application No. 20040030258 to True-Test Limited discloses a flexible, conformable, sensor assembly that includes an electrode array especially adapted for stable, long-term recording of EEG signals from a pre-term or neonatal infant in intensive care.

Grossman, T., The Development of Emotion Perception in Face and Voice During Infancy, Restorative Neurology and Neuroscience, Vol. 28 (2010): 219-236, provides a literature review of how the ability to interact with others develops during infancy and what brain processes underpin infants' perception of emotion in different modalities. Grossman discloses a set of electrophysiological studies that provide insights into the brain processes underlying infants' developing abilities.

US2015018705A1 describes a neural analysis and treatment system that includes a computing device with a memory for storing an application that is executable on a processor to receive amplitude-integrated electroencephalography (aEEG) and range-EEG (rEEG) measurements associated with a patient.

US2014128763A1 describes an evoked response testing system for neurological disorders.

US2013060158A1 describes a method of identifying a site of injury in a pediatric patient with brain injury which comprises obtaining an electroencephalogram signal on the patient.

US8805489B1 describes a method and system for identifying personal meaning of an auditory stimulus to the person, such as, but not limited to, passion, using the measurement and processing of EEG and the identification of the presence of specific patterns in the signal.

Available mechanisms have a variety of limitations. Consequently, it is desirable to provide improved mechanisms for collecting EEG data from infants and young children.

The disclosure may best be understood by reference to the following description taken in conjunction with the accompanying drawings, which illustrate particular embodiments.

### BRIEF DESCRIPTION OF THE DRAWINGS

Figures 1A and 1B illustrate examples of a system for performing data collection and functional interpretation of underlying brain processes of infants and young children.
Figures 2A and 2B illustrate examples of electrode configuration for optimum data collection.
Figure 3 illustrates examples of EEG features that can be observed using the data collection system.

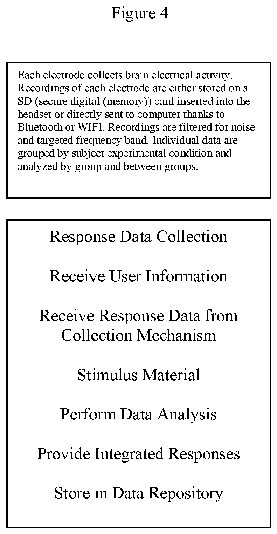

FIGURE 4 illustrates one example of a technique for performing data synchronization.

### DETAILED DESCRIPTION OF PARTICULAR EMBODIMENTS

From birth, infants develop and show their ability to experience and express different emotions. They express biologically basic emotions such as contentment when they are fed or distress when they need attention. Considering the significant role that emotions have in overall brain functioning, research on the nature of infants' emotion has generated considerable interest in recent decades. Currently, this interest is focused on core brain patterns associated to infants' emotional perception and development.

For decades, emotional expressions have been studied in normative infants' population noting that facial components of emotional expression are present very early after birth (Camras, Holland and Patterson, 1993; Calhoun and Kuczera, 1996; Izard & Malatesta, 1987; Phillips, Wellman and Spelke, 2002). If we consider facial expressions as external cues representing an internal emotional state then, a following question is, do infants experience emotions in the same way as children or adults? From a variety of studies, we know that infants are not aware of their own emotional states before 2 years of age. Infants experience what is known as "dissociation between state and experience" (Lewis and Michalson; 1983; REF) meaning that infants have the emotion but not the experience of the emotional state. The ability to experience our own emotions depend on the development of a number of cognitive skills that do not appear until the second year of life. One of these cognitive skills is the so-called "self-consciousness" (also called the sense of self) and its emergency is not automatic but rather, is the product of a maturational process of brain structures (mainly prefrontal cortex, limbic cortex, basal forebrain, amygdala, hypothalamus and brainstem) continually changing from birth and over the different stages of infancy. Thus, until infants acquire such level of neurodevelopment, the ability to experience any emotion as an own conscious state is not present (see Lewis, 2003; 2011).

Thanks to the latest advances in neuroimaging, we can investigate deeper the neural mechanisms associated with early affective states in terms of structure maturation and synapse formation. Studies using high spatial resolution techniques such as Functional Magnetic Resonance Imaging (fMRI) have largely documented the cortical and subcortical structures (e.g., mainly amygdala, anterior insula, medial prefrontal cortex) involved in processing specific emotions like fear or affective communication over the first year of life (Callaghan et al., 2014; Johnstone, 2006; Morgane et al., 2005). However, fMRI lacks the temporal resolution needed to capture continuing neural functioning changes associated to emotion processing (Banaschewski and Brandeis, 2007; Hajcak, Moser, & Simons, 2006). Instead, EEG technique is particularly accurate to investigate cognitive processes that occur on the milliseconds' timescale given its high temporal resolution (de Haan, 2007; REF). Such level of time accuracy is critical when exploring month-to month changes in brain-behavior relations (Cuevas and Bell, 2011). Furthermore, to measure emotional-related activity in young infants, EEG has many key strengths compared to other neuroimaging methods. One of these advantages is that it allows to collect brain-related data in a relatively unconstrained experimental environment compatible with infants' ongoing behavior. Moreover, the use of high-density EEG allows for greater spatial resolution which is critical when making comparisons across brain hemispheres (e.g., asymmetry) and scalp sites. Overall, EEG method has the advantage of being objective and not subject to an observational scoring metric.

Electroencephalogram (EEG) is a research method with a large history and promising future to continue advancing the study of infants' emotional development.

### 2. Measurement of emotions with EEG in young infants (split by age)

Although there is considerable growth of infants' emotion research with EEG methods (see latest journal publications: Gomez et al., 2016; Porto et al., 2016; Taylo-Colls and Pasco Fearon, 2015; Van den Boomen, Munsters and Kemmer, 2017), data often appears disperse and full of uncertainties related to neural signatures underlying affective processing in young infants and children. Mostly, this lack of consistence is centered on type of emotions being measured (i.e., basic or derived), age factor and methodological aspects like appropriate EEG testing paradigms.

A general question when doing EEG research in infants refers to the correct frequency identification. According to research, the normative dominant frequency bands in infant EEG are 4-6 Hz and 6-9 Hz (Calkins, Fox and Marshall, 1996; Marshall, Bar-Haim and Fox, 2002). These are slow wave brain that is thought to index infants' neural activation (Allen, Coan and Nazarian, 2004; Davidson, 1988). However, data often appears disperse and full of discrepancies related to frequency band boundaries. Mostly, this lack of consistence is centered on type of emotions being measured (i.e., basic or derived), age factor and methodological aspects like the more pertinent EEG approach. Also, infants' EEG is sensitive to recording context which means that changes in EEG are dramatically influenced by the content of sensory input, the stimuli valence (Davidson and Fox, 1982; Missana and Grossmann, 2015), action observing (Cuevas et al., 2014; Fox et al., 2016; Marshall and Meltzoff, 2011) and maternal psychological background like for instance depression (Field et al., 1995; Jones et al., 1997; Lusby, Goodman, Bell and Newport, 2014).

Because brain development is a dynamic system, unstable patterns of neural activity are expected throughout different age periods. Indeed, it has been shown that early emotional stimulation correlates with dramatic changes in infants' EEG response. According to reviewed literature, the neural signatures of positive affect development over the first year can be explored within two periods of age. **In** the next section, we provide a review of latest EEG research insights in the field of infants' emotion during the first year of life based on aged-related critical changes in their brain activity. Furthermore, we will discuss the pros and cons of EEG methods currently being applied to the study of emotion-processing in clinical and non-clinical infants' population.

### Age window: 0-6 months

### Core emotions

From a biological point of view, the emergence of affective networks is a sequential process since development of certain emotions (fear, sadness, guiltiness, empathy, frustration etc.) has necessary to precede others (distress, comfort, interest). Since birth (0-2 months), infants show an innate preference to facial expressions and affective voices. Currently, we know from neuroimaging research that newborn brains can discriminate basic emotions when presented with facial expressions such as happy and sad (Johnson, Dziurawiec, Ellis, and Morton, 1991) or voices with different emotional valences (Cheng et al., 2012; Grossmann et al., 2005; Zhang et al., 2014). Precisely, face-specific activity in the infant brain appears about 2-months onwards (de Heering et al., 2005, 2012) but this not necessarily entails emotion recognition or processing as occurs in adults. A preceding maturation of limbic structures (amygdala) will be necessary as well as a complete development of the visual system to enable an adult-alike emotional processing system.

With the development of the visual representation areas of the cortex, the infant's limited sight will quickly develop into full binocular vision. According to literature, early facial perception bias is considered a foundation of emotion-processing related network (Leppänen and Nelson, 2008) since human faces have associated biologically relevant features (e.g., faces can indicate a threat or well-being).

Between the ages of 3-6 months, infants' capacity to recognize emotions improves dramatically co-occurring with significant growth of limbic structures related to recognition memory (i.e., hippocampus) and key neural programs deployment (i.e., exuberant period). At this age, infants give smiles and laughs (smiling before that age is considered reflective, not emotional) showing excitement when they see or experience pleasant events; they cry when upset, seek for comfort and are able to appreciate differences between adults based on the way they look, talk or feel.

**In** the following section we will describe infants' EEG characteristics as a function of their neurodevelopment milestones.

### Main EEG developmental changes

Within the period from 0 to 5 months, is important to identify the typical EEG signatures and frequency band changes associated with infants' emotional behavior. Various studies have shed light on how the EEG signal varies according to different developmental stages. For instance, we know that typical alpha oscillations (power spectrum between 7 and 15 Hz) is not present before age of 10 months (REF). Instead, infants exhibit the so-called posterior "alpha-alike" activity (oscillation range of 8-13Hz) resembling the classical alpha rhythm observed in adults (Lindsley, 1938, 1939; Smith, 1938, 1941). Changes in these posterior rhythms may occurs in short periods of time shifting from 3-9Hz at 1 week of age to 4-9Hz. By the age of 3 months, the primary peak is at approximately 5Hz (Diego, Jones and Field, 2009).

In early development studies, lower frequency EEG activity in the theta band is related to cognitive and emotional processing in young infants (Futagi, et al., 1998; Kugler and Laub, 1971; Lehtonen et al., 2002; Paul, Dittrichova and Papousek, 1996). Age-related increases in power in infant theta band have been reported from 3 to 6 months of age (Lehtonen et al., 2002;Stroganova and Orekhova, 2007). In some of these studies, theta activity has been associated with infants' early expression of positive and negative emotions like comfort or drowsiness (Futagi et al., 1998). The specific theta frequency range reflecting the emergence of these affective states, is susceptible of variations due to the neurodevelopmental events. For instance, we know that infants' theta frequency range used as reference is approximately 3-6 Hz but these boundaries are inconsistent across studies making difficult to find an accurate interpretation of the functional significance of the EEG spectra. Therefore, in light of the observed inter-individual variability in infants' EEG bands, assigning frequency intervals to specific oscillations can sometimes be misleading and therefore, an age-adjustment calibration should not be arbitrary.

### EEG studies and methods

The most salient signal of early emotion processing in infants is their attentional sensitivity to human faces. The reviewed evidence indicates that infants start to exhibit some degree of face-processing around 2-months-old (Easterbrook, Kisilevsky, Muir and Laplante, 1999; Walton & Bower, 1993). Indeed, neuroimaging data indicates that subcortical structures, like the amygdala, are functional at birth thus, playing a critical role in newborns early attentional bias toward faces (Johnson, 2005; Leppänen and Nelson 2009). However, the development of more advanced face-emotion discrimination skills appears after a greater exposure to social interactions (Le Grand, Mondloch, Maurer and Brent, 2001; Pascalis et al., 2005)
Concerning to the neural correlates of facial-emotion processing, Event-related potential (ERPs) studies show a different pattern of neural activity in response to presentation of faces compared to non-face objects in infants from 3-months of age (Halit, et al., 2003; 2004). However, unlike adults, it seems that the same infant's face-processing mechanisms are activated when presenting a broader range of other stimuli (i.e., objects, animals) suggesting that underlying networks become more specialized to human faces over the course of development (Halit et al., 2003).

**In** newborns it is quite difficult to precise the level of attentional engagement to visual emotional elicitors like faces, since connectivity patterns of attentional networks are still immature. Furthermore, the ability to recognize different facial expressions identities are not present until the age of 5-7 months. Conversely, the auditory system is much more developed at birth than the visual system and can provide better EEG insights of emotional discrimination (Carral et al., 2005; Gottlieb, 1971; Háden et al., 2009; Trehub, 2003; Ruusuvirta et al., 2003). Therefore, to investigate emotion perception in young infants, instead of using visual stimuli such as facial expressions, is more convenient to use a multimodal approach. For instance, facial and vocal stimuli synchronization to measure emotional discrimination.

**In** view of the above, in young infants is feasible to address emotional processing EEG-related activity by employing classical auditory-based experimental paradigms. The Mismatch Negativity (MMN) is especially adequate in newborns as it can be elicited in the absence of attention (Näätänen et al., 1993; 2007; Luck, 2005). Also, a modified auditory odd-ball paradigm is commonly used to measure neural response to affective voice in neonates. **In** this regard, few studies have identified the neural correlates of emotional voice processing in neonates using respective paradigms also combined with an ERPs approach (Cheng et al., 2012; Zhang et al., 2014). **In** one of these studies, it was shown that affective voices of fear and anger, elicited a different pattern of neural correlates (e.g., mismatch response about 150-250ms post-stimulus) in neonates (Zhang et al., 2014). Authors concluded that the early brain may have different neural mechanisms to process fear and angry vocal expressions although they agree that this early discrimination can't be conceptualized as newborns having the emotional experience of fear or anger. More likely, it can be interpreted as a preceding neural endowment for later emotion skill development.

### Discussion

One main questions in the study of neural basis of emotions in young infants (mainly before the age of 6 months) refers to which specific emotions are feasible to be studied with a minimum of reliability. Often researchers focus on observable displays of affective expression to interpret infants' emotions. Although decoding facial and body expression provide insights of identifiable emotions, researchers differ on the range of emotions that infants can experience (see Camras, 2004). **In** general, the emotional state in newborns has been defined as a continuum of mood variations going from distress to comfort and vice versa, being highly affected to caretakers' own states (i.e., they become irritable when mother is irritable). However, the neural correlates of early fear or anger has been studied in young normal neonates (Cheng et al., 2012; Zhang et al., 2014) and the results are not conclusive. Indeed, converging evidence show that the attention bias towards threat-related stimuli appears at age 6-7 months corresponding to the developmental period when fear emerges (Leppänen and Nelson, 2012). Thus, spontaneous anger or fearful facial responses in newborns may reflect the emergence of the neural circuitry for processing more refine affective stimuli with socio-emotional relevance but does not indicate the actual experience of anger or fear. Furthermore, we find that disentangling newborns' emotion states based their facial expression correlates are not sufficient given that infants at this age show a high capacity to imitate adults (Heyes, 2011; Isomura and Nakano, 2016; Oostenbroek et al., 2016). An optimal way to explore infants emotional experience (i.e., anger, comfort, enjoyment) using EEG could be by eliciting situations that interrupt or increase instrumental actions such as sucking, feeding or audiovisual contingencies (i.e., goal blockage paradigm; Lewis et al., 1992).

We also find a great deal of EEG studies focused on an emotional valence approach, addressing newborns experience of primary emotions like pleasantness or distress. **In** general, these early-age emotions are explored based differences in frontal right-left regions in response to a variety of positive/negative emotional elicitors (Davidson and Fox, 1982; Fox and Davidson, 1986; Fox and Davidson, 1987). This assumption comes from the EEG asymmetry model wherein a frontal left-sided activation is consistent with positive emotions while a frontal right-sided activation is associated with negative emotions (Davidson, 1993, 2004; Coan and Allen, 2003). Such differential lateralization has been reported in young infants in a limited number of studies although conclusions could be interpreted based on the different stimulus conditions (i.e., novel vs familiar) being sometimes difficult to determine whether these frontal asymmetries are reflecting emotional processing activity or perceptual-related biases. Therefore, researchers should keep in mind that measuring frontal EEG asymmetry in young infants, frequently entails a high level of fuzziness or inaccuracy due to the neural development factor and a lack of a strong functional significance of EEG frontal asymmetries at that age.

Finally, frontal EEG asymmetries differences are not only hypothesized to be related to early emotional response but also are thought to reflect underlying mechanism of individual's emotional regulation tendencies (Fox, 1994). Thus, such early markers of frontal asymmetries are important precursors to understand individuals' differences in emotional behavior.

### 6-12 months: neural development shift

### Core emotions

A more general question in the field of emotion research is whether specific emotion such as anger or happiness can be identified based on a particular pattern of brain activity. **In** infants, this question has a growing interest given that neural response could serve as an early development marker to predict infants' future socio-emotional skills (Fox et al., 1995; Harmon-Jones et al., 2010: Paulus et al., 2013; Tomarken et al., 1992; Reid et al., 1998).

With the course of brain development, more refined emotions arise as a response to a more sustained environment stimulation. By age of 6-month onwards, other observable facial expressions (laughing, disgust, fear, etc.) accompanied with body movement sequences (shaking arms, legs) are exhibited suggesting states of surprise, interest, joy, anger, fear and disgust (Lewis, 2007). Some of these emotions (i.e., fearfulness, surprise) may reflect underlying cognitive processes such as perceptual discrimination, short-term memory or socialization development. For instance, converging evidence show that attentional bias to fearful faces (appearing around 6-7 months of age) correlates with different neural responses compared to happy faces (Jessen and Grossmann, 2015). This differentiation does not occur in younger infants who pay greater attention to smiling faces instead, indicating an enhancement of the cortical response after 6 months of age (Bayet, Quinn, et al., 2015; Jessen and Grossmann, 2014, 2015; Leppänen and Nelson, 2009, 2012; Yrttiaho et al., 2014). Between ages 5 and 6 months, infants begin to show stranger anxiety and they will show discomfort or more distress visibly.

Towards the end of the first year of life, infants become highly emotional going from intense happiness to intense frustration or anger quickly. They also become start to become aware of other peoples' emotions showing a subtler understanding of emotional communication cues like for instance, production of anticipatory smiling as well as managing contextual emotional cues during perception of facial expressions (Venezia, Messinger, Thorp and Mundy, 2004). Around age of 12 months, infants begin to make the connection between facial expressions with specific emotions thus, exhibiting a rudimentary awareness of their own emotional states.

### Main EEG changes

**In** general, changes in EEG spectral power due to neural development are expected with a relative reduction of low-frequency rhythms (i.e., delta waves 0.5-4 Hz). By 6 months of age, a dominant Theta oscillation (3-6 Hz) widely distributed over frontal and posterior scalp regions is elicited in response to variety of emotional elicitors and behavioral context including feeding, drowsiness, social interaction or cognitive activity (e.g., anticipation).

**In** early research, a theta frontal topographic distribution has been related to a higher contribution of attentional networks (Orekhova et al., 1999; Stroganova, Orekhova, and Posikera, 1998; Orekhova et al., 1999). **In** one of these studies, the frontal theta activity of an anticipatory period of the peek-a-boo game was compared to periods of quiet attention (i.e., watching soap bubbles) in different groups of age (7-8 and 9-11 months). Authors reported differences in theta power (assessed in the range of 3.6-4.8Hz) with a relatively greater amplitude observed among the younger group of age (Orekhova et al., 1999; Stroganova and Orekhova, 2007). This is consistent with undergoing maturation of infants' executive cortical networks and with other EEG studies reporting greater frontal theta synchrony (over 8 months of age) indexing sustained attention (Bazhenova et al., 2007; Uhlhaas et al., 2010). However, it is not clear yet whether there is an increase in theta amplitude in other tasks. Unlike the infant alpha rhythm, research on developmental changes in theta frequency during infancy are scarce.

Further, with developmental changes in the frequency spectrum and synchronization of neural oscillations, asymmetrical brain activity becomes a more reliable index to measure positive-negative affective processing (Fox et al., 1992; REF). Based on Davidson and Fox studies (1982; REF), this lateralized EEG activity seem to be stable by age of 10 months consistent with a greater exhibition of social and emotional behavior. Indeed, convergent evidence show that EEG right-sided frontal asymmetry is associated with behavioral inhibition and social withdrawal in infants while left frontal asymmetry is linked with trait tendencies toward a general approach, or positive emotion (Fox and Henderson, 1999). Therefore, most of the reviewed research characterizing the neural patterns of affective behavior in this group of age, are focused on EEG asymmetries in the range 3-12 Hz.

Although these asymmetries are currently the most powerful indicator of infants' affective states, nonetheless, the assumed age stability is not clearly defined across different studies. For example, Vuga and colleagues (2008) reported long-term frontal asymmetry stability across 6-36 months interval while Jones and colleagues (1997) across 24-30 months. More recent research showed moderate stability between 10 and 24 months (Howarth, Fettig, Curby and Bell, 2016). These discrepancies make difficult to precise the functional nature of frontal asymmetries in the study of emotion processing and development in infants.

### EEG studies and research methods

Based on reviewed literature, we have differentiated two lines of research to focus on. One refers to neural patterns associated to emotion recognition. Thus, here the main question is how infants perceive and recognize other people' emotions as well as how their emotional perception develops. A second line of research interest investigates the EEG markers of differentiated affective states, expressed in infants as specific mood states such as comfort, happiness, distress, sadness, engagement, etc., in response to an evocative elicitor or situation (new toy, mother approach) that further, can be modulate (or not) by a neural marker trait. **In** next lines, we describe main EEG evidence of affective processing in infants following mentioned above lines of research.

### First line of research: ERPs and frontal asymmetry

Exploring infants' emotion across modalities can be relatively less fuzzy with the course of development given the enhancement of visual attentional circuitries functioning. This allow researchers using higher number of trials which is of special interest considering the generally low signal to-noise ratio in infant ERPs studies. Furthermore, capturing infants' attention during longer time is less challenging after age of 6 months.

A large deal of research methods to investigated infants' responsiveness to emotions are based on ERPs approach. A simplest assessment consists in recording ERPs activity while infants watch pictures of a happy and fearful faces compared to pictures of objects. Extend research exploring face-sensitive ERPs in infants, has identified several components (i.e., N170, N290, Nc, P400) presumably associated to a variety of salient facial-emotion expression (Allison et al., 1994, 1999; Batty and Taylor, 2006; De Haan and Nelson, 1999; Nelson and De Haan, 1996; Kobiella et al., 2007). The component Nc occurring between 400 and 800ms, is one of the most studied components in infant ERP research. This component has a negative deflection and is mostly distributed over frontal and central electrode sites. Although is not exclusively elicited in response to face expressions, reviewed studies show that topography and temporal characteristics differed from those associated to perception of objects in infants (de Haan and Nelson,199 9; Taylor, McCarthy, Saliba, and Degiovanni, 1999).

Attentional sensitivity to angry faces compared to pain and fearful faces been studied in a 7 to 8-month-old population based on an ERP and frontal asymmetry approach (Missana, Grigutsch and Grossmann, 2014; Kobiella, Grossmann, Reid and Striano, 2008). In one of these studies, the Nc ERP component was reported in response to emotional expressions with a greater enhancement to angry expressions. In this context, the Nc has been interpreted as reflecting infants' allocation of attention. When comparing infants' pattern of frontal asymmetry to adults' pattern in response to angry faces, results showed opposite frontal asymmetries: greater relative left frontal activation in adults (related with an approach tendency) while a greater relative right-frontal activation was shown in infants suggesting a motivational tendency to avoidance.

Also, the N290 appears by second half of first year, indexing infants' facial expressions discrimination based on social cues and not just on a negative-positive valence dichotomy. For instance, it has been shown that an angry expression elicit a larger N290 amplitude compared to a fearful expression. Even though both emotions are negative, the angry expression is thought to cause a more discomforting reaction compared with the fearful expression thus, suggesting discrimination process of social cues (Kobiella et al., 2007).

Neural underpinnings of infants' emotion processing across sensory modalities (visual-auditory-haptic) is a question of great research interest. Very few EEG studies explore emotional processing using multimodal experimental designs (Grossmann et al., 2006; Friedrich and Friederici, 2005). For example, in one of these studies, ERPs indexing processing of emotionally congruent and incongruent face-voice pairs was investigated in a 7- month-old infants' population. Findings revealed a distinct pattern of ERPs (mainly Nc and Pc) as a function of cross-modal emotional congruity (Grossmann, Striano, and Friederici, 2006). Thus, indicating that emotional information integration across modalities occurs at relatively early in life.

**In** summary, an ERP approach may offer different research directions to answer questions of emotion processing and development in human infants. However, a main limitation is that unlike adults, infants do not show well-defined peaked ERP responses. Instead, they exhibit greater slow wave activity mainly during the first two years of life which is indicative of poor synaptic efficiency. As brain response consistency increases with age, well-defined ERP waveforms are expected to emerge more clearly around 4 years (Nelson and Luciana, 1998; Taylor, Batty, and Itier, 2004). ERPs studies before that age must be conducted under high levels of experimental control which sometimes interferes with a desirable ecological assessment of emotions.

### - Second line of research:

In the course of development, infants express emotional states through a visible repertoire of facial expressions increasingly resembling the way adults do. They smile to communicate happiness and cry to express disgust or discomfort. Furthermore, infants already show "affective styles" in response to different evocative stimuli or events varying in intensity and duration (Davidson, 2003, 2004). Such affective propension has been linked to specific patterns of frontal EEG characterized as early traits of infants' emotional abilities. Of the emotions examined at this interval of age, happiness, fear, sadness, and disgust, have received more attention (compared for instance, with anger) which is relatively easier to evoke in the laboratory using established norm-stimuli (e.g., pictures, facial expressions, films).

In general, the pattern of frontal EEG asymmetry indicates infant's disposition or bias, toward withdrawal-approach behaviors (i.e., Fox, Henderson, Rubin, Calkins and Schmidt, 2001) probably reflecting the activity in the amygdala (Davidson, 2000). In resting baseline, EEG frontal asymmetrical may be an important neuromarker to predict infants' emotional responses to evocative stimuli or events (Wheeler et al., 1993). For example, there is data showing that infants with resting right frontal asymmetry tend to cry more during maternal separation (Davidson and Fox, 1989; Fox et al., 1992) So far, frontal asymmetry is the most frequent approach to study infants' affective traits or capabilities (Coan and Allen, 2004; Davidson and Fox, 1989; Davidson, 1992; 1993; Fox, 1991, 1994).

Consistent with referred frontal asymmetry models (approach/withdrawal and positive-negative valence; Davidson 1993; 2002) resting frontal EEG activity is hypothesized to reflect a predisposition to positive versus negative affect and future infants' ability to regulate emotions. Part of the interest on this line of research, lies on its potential clinical application to predict infants' predisposition to certain affective disorders or styles. Indeed, hemispheric frontal activity during resting states is considered an early neurophysiological marker of depression risk (Allen and Reznik, 2015). For instance, there is large amount of data showing greater relative right-frontal EEG asymmetry during resting state periods in infants (e.g., 10-months-old) of depressed compared to non-depressed mothers (Dawson et al., 1992; Diego et al., 2006; Field et al., 1995; 2000; Field and Diego, 2009; Jones et al., 2001; Jones, Field and Almeida, 2009). Other studies exploring neural markers of separation anxiety also found a greater right-sided frontal activity at resting state in infants who cried in response to maternal separation compared to those who not cried (Davidson and Fox, 1989).

The frontal asymmetry elicited during a resting state period can be altered in response to a variety of emotional elicitors. Thus, a feasible way to predict infants' state changes in emotion is by measuring the impact that approach-oriented (i.e., affective touch, familiar voice) versus withdrawal-oriented (i.e., a stranger person, intrusive interaction) stimuli has on frontal activity (Coan et al., 2001; Coan and Allen, 2004; Ekman et al., 1990; Davidson and Fox, 1982). A simultaneous measurement of physiological arousal (heart rate) and attention engagement correlates (gaze duration and shifts) using appropriate paradigms (i.e., visual discrimination, toy social referencing, cross-modal transfer) is highly recommended especially when the main research goal is to evaluate affective state changes as result of evocative stimuli.

Additionally, consideration must be given to the type of emotionally evocative stimuli used to investigate frontal asymmetry patterns. It might be reasonable to expect individual differences in the frontal asymmetry activity affecting infants' response to the same evocative elicitor (Davidson and Fox, 1989). However, depending on the objectives of the study, one may adopt an individual differences approach or a normative approach. Thus, the former recommendation applies to studies focused on exploring patterns of cortical activation asymmetry as a stable trait to predict emotional reactivity or motivational tendency to evocative stimuli. For instance, the predisposition of infants to cry or not in response to caregiver separation or to smile or feel shy in the presence of a stranger person can be predicted based on their individual frontal asymmetries patterns. Within this approach, is recommended to add a longitudinal component to investigate the impact of early positive and negative affectivity experiences on development (Calkins et al., 2002; Coan and Allen, 2003; Davidson, 1998; Gaertner et al., 2008; Hill-Sonderlund and Braungart-Rieker, 2008; Kochanska and Knaack, 2003; Wheeler, et al., 1993). Conversely, a normative approach applies to those studies more focused on measuring infants' frontal asymmetries associated with affective states elicited in controlled environmental-experimental conditions (Buss et al., 2003; Cattell and Scheier, 1961; Diaz and Bell, 2012; Fox et al., 2001; ). Examples of this would include infants' differences in frontal asymmetry resulting from presentation of happy-sad facial expressions, stressful situations like stranger approach or different types of novel stimuli.

**In** addition, when looking at frontal activity asymmetries in non-clinical population, the combination of EEG recordings with other techniques such as simultaneous facial coding may add further insights into infants' categories of emotional states. The facial coding approach includes examination of right and left EEG patterns during baseline and emotion-eliciting conditions (i.e., peek-a-boo game) while simultaneous coding infant's affect (facial expressions) for example as positive, negative, and neutral. **In** this regard, there is evidence showing a distinct and independent pattern of left-sided frontal activation mirroring two types of infants' smiles: in response to mother vs in response to a stranger approach (Fox and Davidson, 1988; see also Field et al., 2011; Jones et al., 2001). Moreover, the functional significance of infants' transitory theta rhythm has been addressed using a simultaneous EEG-videotaped method (Dawson et al., 1992; Field et al., 2011; Futagi et al., 1998). **In** one of these studies, it was demonstrated that frontal and posterior patterns of theta rhythms are associated with emotional reactions such as crying and sucking, also with other cortical activity such as gazing and handling (Futagi et al., 1998).

Finally, considerations should be given to the used montage-reference site when measuring frontal asymmetries. Traditional sites refences (i.e., Cz vertex, mastoids) are problematic when the EEG is measured based on power spectra (see Pivik et al., 1993; Hagemann et al., 2001). Therefore, the main suggestion is to use a recording reference scheme sensitive enough to account for individual variations in frontal asymmetry. We refer reader to EEG literature to find further details on reference-site guidelines (Hagemann, 2004; Nunez et al., 1994; Pivik et al., 1993).

Overall, frontal asymmetry is presented in infants' literature as an advantageous approach to measure emotions at early stages of development. However, we consider that there are still inconsistencies likely due to differing theoretical and empirical grounds. **In** next section we discuss these discrepancies and provide some suggestions to future research.

### Discussion

Most of the reviewed studies agree that asymmetric patterns of activity in frontal sites may serve as a neural marker of infants' affective response. We also have reviewed that this frontal asymmetry which starts to appear stable around 10 months of age, correlates with motivational properties of affective states (approach-withdrawal behavior) as well as with the experience and expression of positive/negative emotions (Dawson, 1994; Diaz and Bell, 2012; Fox, 1991, 1994). This is not to say that the frontal asymmetry is causally involved in the production of these emotional responses since data supporting this assumption in infants is mostly based on correlational evidence and hence, subject to alternative explanations. For instance, a pattern of frontal activation may also reflect functional developmental changes on relevant neural circuitries (i.e., Alerting Attention Network) or the influence of rewarding values of the stimuli.

Furthermore, although the number of studies supporting this direct association is sufficiently large in adults' literature, in infants' research we found that the asymmetrical frontal activity and respective emotion experience and expression is not sufficiently addressed beyond the context of emotion recognition (e.g., mainly based on visual discrimination of facial expressions). The amount of EEG studies exploring the relationship of frontal asymmetries with changes in emotion states is even less in non-clinical population.

A second part of this discussion refers to the influence of individual differences in EEG frontal asymmetry activation and its functional meaning in a non-clinical research context. When measuring cortical asymmetries in infants either to make inferences of their emotional state in relation to evocative stimuli or to predict their affective traits, there are sources of variation to consider mostly related with individual differences. Regarding to infants' trait asymmetries hypothesis, the high heritability of the EEG spectra makes difficult to precise whether frontal asymmetry derives from a genetic predisposition or from environmental influences (Coan, 2003; MacDhomhail et al., 1999; Lykken et al., 1982). In both cases, is recommended to strengthen methodological aspects to ensure the reliability of their measures (e.g., increase the sample size, resting time, appropriate montage-reference schema). For instance, measurement of the resting frontal asymmetry consistency across multiple sessions, can help researchers to differentiate changes due to individual stable trait from changes in response to specific experimental manipulations (see Hagemann, 2000). Within the same experimental session, several measurements of resting frontal activity might increase data reliability but implies facing certain drawbacks. In this regard, we noticed that most studies only conduct a single measurement of frontal asymmetry in the resting state or baseline condition. Usually, this is because is very difficult to keep infants still in the lab for long periods without undesirable mood changes affecting the recordings.

In addition, to increase the internal validity of the study and avoid possible confounding, latest studies reports compare mother/infant frontal asymmetry similarities during emotionally salient situations (Collan and Allen, 2004; Jones et al., 2001; Atzaba-Poria, Deater-Deckard, Ann-Bell, 2017). **In** this regard, it may be also convenient to relate frontal EEG responses to concurrent measures of maternal influencing behavior (i.e., positive vs negative affect) to determine if the functional significance of EEG frontal asymmetry is grounded in motivationally-specific factors or a biological trait (LoBue, Coan, Thrasher, DeLoache, 2011; Wen eta al., 2017). Finally, in using frontal asymmetry changes as a function of specific evocative stimuli conditions, is recommended to control individual differences due to age as it may explain typical discrepancies in EEG power variations in relation to specific emotional elicitors.

At some point, we find the frontal asymmetry formulation pointing toward an excessive oversimplification in infants' research, due in part to some data inconsistencies but also to a lack of precise definition of what the emotional valence and motivational frontal asymmetrical activity might be in infants less than one year old (in contrast to adults). For instance, in infants (4-9 months of age) is not clear yet the association of right-frontal EEG asymmetry with anger as some of reviewed research confounded the valence of the emotion (positive-negative) with the direction of motivation (approach-avoidance), making difficult to discriminate which of those (or a combination of both) the frontal asymmetry reflects (Davidson and Fox, 1989; Harmon-Jones, 2004; He, Degnan, McDermott et al., 2010). Thus, it remains of great importance for future research to detect the conditions under which frontal asymmetry is the most appropriate approach to study changes in emotions during evocative task or stimuli. As an alternative, we found promising the capability model proposed by Coan and Allen (2006) wherein individual differences in frontal asymmetry are predicted to be more pronounced during an evocative task or emotional stimulation rather than during resting state.

### 3. General consideration: current perspective and research directions

In this review, we have highlighted the most important advances and theoretical perspectives regarding infants' emotion behavior as a function of their neurodevelopment stage. Further, we have discussed the main advantages and disadvantages of using EEG to investigate infants' emotions and strength and weakness of current outcomes.

The interest of EEG methods in the study of infants' affective development during the first year, has increased notably during the last decade. Despite EEG lacks accurate high-resolution brain images, it provides valuable tools in assessing the neural underpinnings features associated to human affective development during the first years of life. Generally, ERPs and power spectrum measurements have become essential to investigate the infants' cognitive neuroscience. Other neuroimaging methods like source localization, could help further to clarify the interpretation of ERP or frequency band spectrum but it is quite difficult mapping these activations onto brain images collected by structural MRI. This is almost impracticable in non-clinical research with ages under 36 months. Alternatively, magnetoencephalography (MEG) is an interesting technique to study emotions given its signal localization high-accuracy compared to EEG. However, although it can be used with infants well, entails a higher cost and technically is more challenging than EEG (see Imada et al., 2006).

A combination of EEG with other neuroimaging techniques has become a common practice to study cognitive and affective domains. For instance, the simultaneous recording of EEG and functional near-infrared spectroscopy (fNIRS) is a suitable method for measuring the role of prefrontal cortex activation in emotion processing (i.e., Nishitani, Doi, Koyama and Shinohara, 2011). Such coregistration is practicable in infants as requires less cooperation from their side (e.g., Koch, Steinbrink, Villringer and Obrig, 2006). Specially promising is the hyperscanning technique for understanding mother-infant inter-brain synchronization during evocative experiences such as emotional bounding, handholding or speech (Hirata et al., 2014).

Reference will now be made in detail to some specific examples of the invention including the best modes contemplated by the inventors for carrying out the invention. Examples of these specific embodiments are illustrated in the accompanying drawings. While the invention is described in conjunction with these specific embodiments, it will be understood that it is not intended to limit the invention to the described embodiments. The invention is set out in the appended set of claims.

### Overview

An efficient and effective system and apparatus for collecting electroencephalography (EEG) data from infants and young children are provided. The system and method use an EEG headset having electrodes positioned at optimum locations.

### Example Embodiments

Consequently, the system and apparatus of the invention use EEG measurements to allow more accurate measurement and monitoring of attention and engagement of infants and young children. According to various embodiments, an EEG headset is provided to subjects for use in a laboratory environment. In particular embodiments, the EEG headset includes multiple electrodes positioned at optimum locations. Data from individual electrodes may be processed prior to continuous transmission to a data analyzer. Processing may include filtering to remove noise and artifacts as well as compression and/or encryption. Individual electrodes are configured to contact the scalp in a variety of areas.

EEG electrodes allow in situ monitoring and tracking of activity including engagement levels. According to various embodiments, the data collection mechanism is synchronized with stimulus material to allow determination of aspects of stimulus materials that evoke particular neurological responses

According to various embodiments, the EEG headset stores data collected from a user exposed to stimulus material for transmission to a data analyzer.

A subject may wear the portable data collection mechanism during a variety of activities. This allows collection of data from a variety of sources while a subject is in a natural state. **In** particular embodiments, data collection can occur effectively in a corporate or laboratory setting.

A variety of neurological, neuro-physiological, and effector mechanisms may be integrated in a data collection mechanism. EEG measures electrical activity associated with post synaptic currents occurring in the milliseconds range. Nonetheless, surface EEG provides a wealth of electrophysiological information if analyzed properly. Portable EEG with electrodes provide a large amount of information.

According to various embodiments, subjects may be exposed to predetermined or preselected stimulus material. In other examples, no predetermined or preselected stimulus material is provided, and a system collects data for stimulus material a user is exposed to during typical activities.

For example, multiple subjects may be provided with portable EEG monitoring systems with electrodes that allow monitoring of activity. Response data is analyzed and integrated. In some examples, all response data is provided for data analysis. In other examples, interesting response data along with recorded stimulus material is provided to a data analyzer. According to various embodiments, response data is analyzed and enhanced for each subject and further analyzed and enhanced by integrating data across multiple subjects.

According to various embodiments, individual and integrated response data is numerically maintained or graphically represented. Measurements for multiple subjects are analyzed to determine possible patterns, fluctuations, profiles, etc.

According to various embodiments, data may show particular effectiveness of stimulus material for a particular subset of individuals. A variety of stimulus materials can be analyzed. According to various embodiments, enhanced data is generated using a data analyzer that performs both intra-modality measurement enhancements and cross-modality measurement enhancements. According to various embodiments, brain activity is measured not just to determine the regions of activity, but to determine interactions and types of interactions between various regions. The techniques and mechanisms of the present invention recognize that interactions between neural regions support orchestrated and organized behavior. Attention, emotion, memory, retention, priming, and other characteristics are not merely based on one part of the brain but instead rely on network interactions between brain regions.

The techniques and mechanisms of the present invention further recognize that different frequency bands used for multi-regional communication can be indicative of the effectiveness of stimuli. In particular embodiments, evaluations are calibrated to each subject and synchronized across subjects. In particular embodiments, templates are created for subjects to create a baseline for measuring pre and post stimulus differentials.

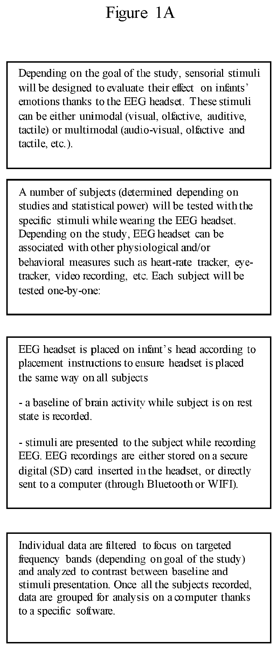

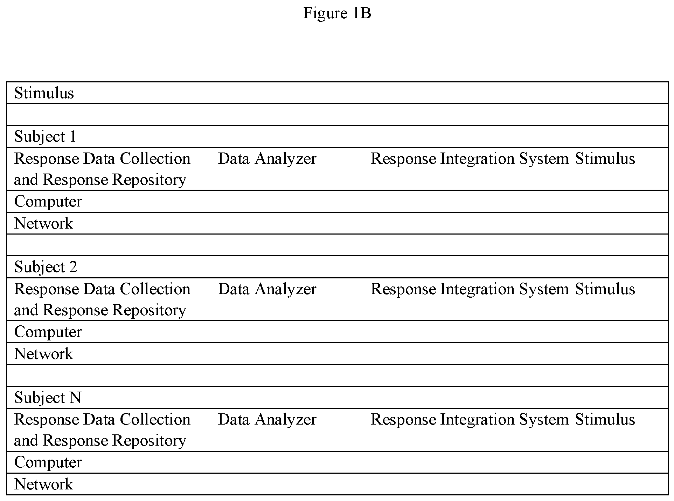

FIGURE 1 illustrates one example of a system for collection of data. Subjects are associated with data collection mechanisms. According to various embodiments, subjects voluntarily use data collection mechanisms such as EEG caps during exposure to particular stimulus materials provided by stimulus presentation mechanism. Data collection mechanisms include persistent storage mechanisms and network interfaces that are used to transmit collected data to a data analyzer. In other examples, data collection mechanisms include interfaces to computer systems that are configured to transmit data to a data analyzer over one or more networks.

Materials eliciting response from subjects may include people, activities, images, personal care compositions, and may involve particular tastes, smells, sights, textures and/or sounds. In some examples, stimulus material is selected for presentation to subjects.

According to various embodiments, the subjects are connected to data collection mechanisms. The data collection mechanisms include EEG electrodes, although in some implementations may also include a variety of measurement mechanisms including neurological and neurophysiological measurements systems such as EOG, GSR, EKG, pupillary dilation, eye tracking, facial emotion encoding, and reaction time devices, etc. According to various embodiments, data includes central nervous system, autonomic nervous system, and/or effector data.

In particular embodiments, data collected is digitally sampled and stored for later analysis. In particular embodiments, the data collected can be analyzed in real-time.

In one particular embodiment, the data collection mechanism includes EEG measurements made using scalp level electrodes.

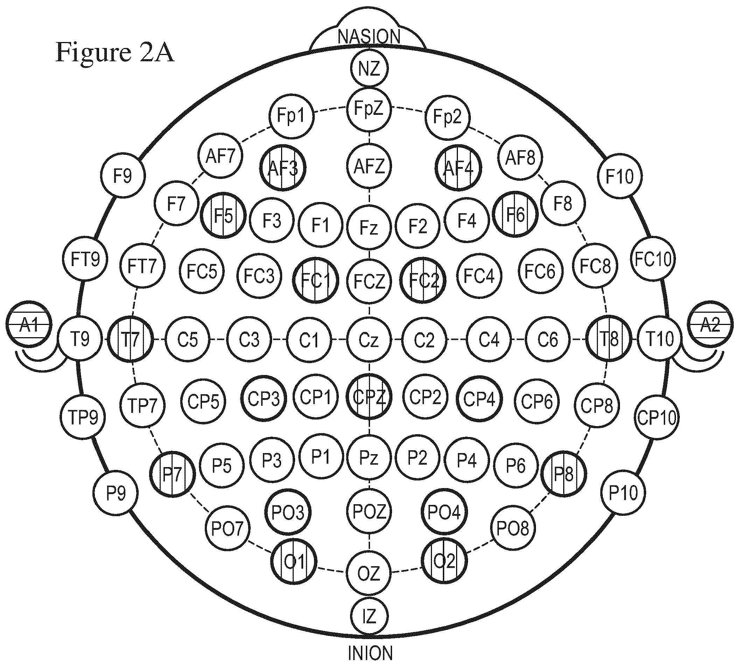

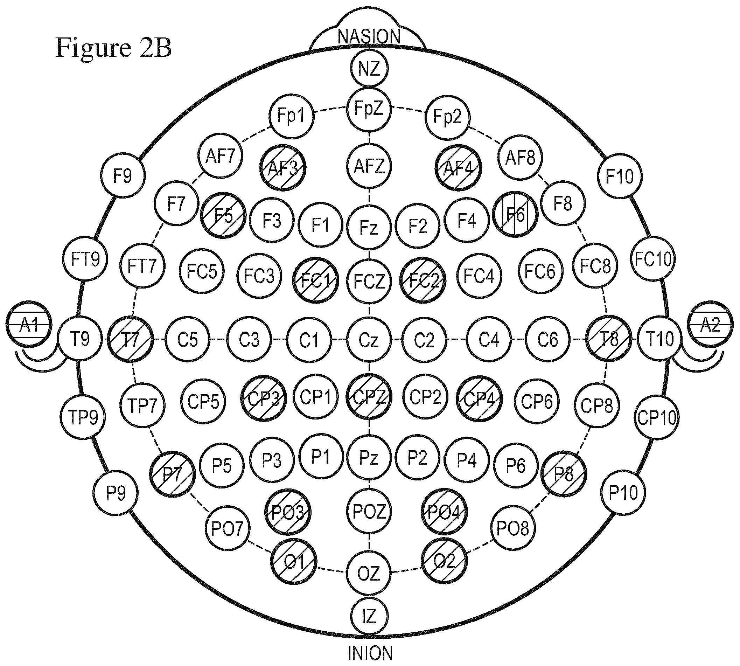

FIGURES 2A and 2B shows a data collection mechanism including multiple electrodes. According to various embodiments, the data collection mechanism is a soft cap having electrodes configured to contact the scalp without the use of electro-conductive gels. Signals may be routed to a controller/processor for immediate transmission to a data analyzer or stored for later analysis. A controller/processor may be used to synchronize data with stimulus materials.

FIGURES 2A and 2B illustrate the data collection mechanism. The data collection mechanism includes multiple electrodes including. It should be noted that although specific electrode arrangement may vary from implementation to implementation, preferred electrode arrangement is provided herein.

Figure 2A shows the electrode (13) arrangement (shaded with vertical lines) for the effective assessment of infants' positive and negative affect with an acceptable level of accuracy. Scalp sites have been chosen to ensure a symmetrical recording in frontal, temporal and parietal areas. Electrodes in the frontal pole (AF3, AF4, F5, F6, FC1, FC2) allow evaluating infants' EEG asymmetries. Temporal electrodes (T2, T3) allow to explore pleasantness-unpleasantness in the auditory domain. Parietal/Centroparietal electrodes (P7, P8, CPz) allow to investigate attention engagement to affective speech and somatosensorial activity. Occipital electrodes (O1, O2) to measure visual emotional processing. A1 and A2 correspond to the reference sites (shaded with horizontal lines).

Electrode position nomenclature template taken from American Electroencephalographic Society guidelines, 1991; Journal of Clinical Neurophysiology, 8, pp.200-201.

Figure 2B shows the electrode (17) arrangement (shaded with diagonal lines) for the effective assessment of infants' positive and negative affect with higher level of accuracy. Scalp sites have been chosen to ensure a symmetrical recording in frontal, temporal and parietal areas. Electrodes in the frontal pole (AF3, AF4, F5, F6, FC1, FC2) allow evaluating infants' EEG asymmetries. Temporal electrodes (T2, T3) allow to explore pleasantness-unpleasantness in the auditory domain. Parietal/Centroparietal electrodes (P7, P8, CP3, CP4, CPz) allow to investigate attention engagement to affective/social speech and somatosensorial activity. Occipital electrodes (PO3, PO4, O1, O2) to measure visual emotional processing and frontal-parietal interplay. A1 and A2 correspond to the reference sites (shaded with horizontal lines). Electrode position nomenclature template taken from American Electroencephalographic Society guidelines, 1991; Journal of Clinical Neurophysiology, 8, pp.200-201.

According to various embodiments, data acquisition using electrodes is synchronized with stimulus material presented to a user. The data collection mechanism also includes a transmitter and/or receiver to send collected data to a data analysis system.

In some examples, the transceiver can be connected to a computer system that then transmits data over a wide area network to a data analyzer.

It should be noted that some components of a data collection mechanism have not been shown for clarity.

Response assessment-based queries may include attention scores, emotion scores, and effectiveness scores. Such queries may obtain materials that elicited particular scores. Response measure profile-based queries may use mean measure thresholds, variance measures, number of peaks detected, etc. Group response queries may include group statistics like mean, variance, kurtosis, p-value, etc., group size, and outlier assessment measures.

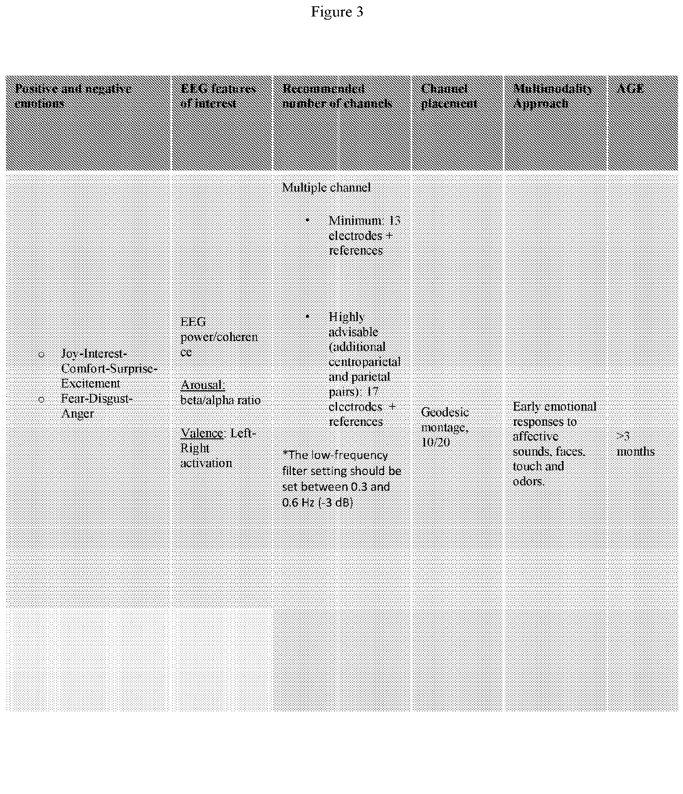

FIGURE 3 illustrates one example of data collection. User information is received from a subject provided with a data collection mechanism. Data is received from the subject data collection mechanism. In some particular embodiments, EEG, EOG, pupillary dilation, facial emotion encoding data, video, images, audio, etc., can all be transmitted from the subject to a data analyzer. In particular embodiments, only EEG data is transmitted. According to various embodiments, response and associated data is transmitted directly from an EEG cap wide area network interface to a data analyzer. In particular embodiments, response and associated data is transmitted to a computer system that then performs compression and filtering of the data before transmitting the data to a data analyzer over a network.

In particular embodiments, the data communication device sends data to the response integration system. According to various embodiments, the response integration system combines analyzed and enhanced responses to the stimulus material while using information about stimulus material attributes. In particular embodiments, the response integration system also collects and integrates user behavioral responses with the analyzed and enhanced response data to more effectively measure and track s to stimulus materials.

Although the foregoing invention has been described in some detail for purposes of clarity of understanding, it will be apparent that certain changes and modifications may be practiced within the scope of the appended claims. Therefore, the present embodiments are to be considered as illustrative and not restrictive and the invention is not to be limited to the details given herein but may be modified within the scope of the appended claims.

### References

A. Porto, J., L. Nunes, M., & Nelson, C. A. (2016). Behavioral and neural correlates of emotional development: typically developing infants and infants of depressed and/or anxious mothers. Jornalde Pediatria. https://doi.org/10.1016/j.jped.2015.12.004
Addabbo, M., Longhi, E., Marchis, I. C., Tagliabue, P., & Turati, C. (2018). Dynamic facial expressions of emotions are discriminated at birth. PLoS ONE. https://doi.org/10.1371/journal.pone.0193868
Allen, J. J. B., Coan, J. A., & Nazarian, M. (2004). Issues and assumptions on the road from raw signals to metrics of frontal EEG asymmetry in emotion. Biological Psychology. https://doi.org/10.1016/j.biopsycho.2004.03.007
Allen, J. J. B., Harmon-Jones, E., & Cavender, J. H. (2001). Manipulation of frontal EEG asymmetry through biofeedback alters self-reported emotional responses and facial EMG. Psychophysiology. https://doi.org/10.1017/S0048577201991255
Banaschewski, T., & Brandeis, D. (2007). Annotation: What electrical brain activity tells us about brain function that other techniques cannot tell us - A child psychiatric perspective. Journal of Child Psychology and Psychiatry and Allied Disciplines. https://doi.org/10.1111/j.1469-7610.2006.01681.x
Blau, V. C., Maurer, U., Tottenham, N., & McCandliss, B. D. (2007). The face-specific N170 component is modulated by emotional facial expression. Behavioral and Brain Functions. https://doi.org/10.1186/1744-9081-3-7
Brooker, R. J., Canen, M. J., Davidson, R. J., & Hill Goldsmith, H. (2017). Short- and long-term stability of alpha asymmetry in infants: Baseline and affective measures. Psychophysiology. https://doi.org/10.1111/psyp.12866
Calkins, S. D., Fox, N. A., & Marshall, T. R. (1996). Behavioral and Physiological Antecedents of Inhibited and Uninhibited Behavior. Child Development. https://doi.org/10.1111/j.1467-8624.1996.tb01749.x
Camras, L. A., Holland, E. A., & Patterson, M. J. (1993). Facial expression. Handbook of emotions*.*
Cheng, Y., Lee, S.-Y., Chen, H.-Y., Wang, P.-Y., & Decety, J. (2012). Voice and Emotion Processing in the Human Neonatal Brain. Journal of Cognitive Neuroscience. https://doi.org/10.1162/jocn_a_00214
Constant, I., & Sabourdin, N. (2012). The EEG signal: A window on the cortical brain activity. Paediatric Anaesthesia. https://doi.org/10.1111/j.1460-9592.2012.03883.x
Cuevas, K., & Bell, M. A. (2011). EEG and ECG from 5 to 10months of age: Developmental changes in baseline activation and cognitive processing during a working memory task. International Journal of Psychophysiology. https://doi.org/10.1016/j.ijpsycho.2011.02.009
Davidson, R., & Fox, N. (1982). Asymmetrical brain activity discriminates between positive and negative affective stimuli in human infants. Science. https://doi.org/10.1126/science.7146906
Davidson, R. J. (1988). EEG measures of cerebral asymmetry: Conceptual and methodological issues. International Journal of Neuroscience. https://doi.org/10.3109/00207458808985694
Diego, M. A., Jones, N. A., & Field, T. (2010). EEG in 1-week, 1-month and 3-month-old infants of depressed and non-depressed mothers. Biological Psychology. https://doi.org/10.1016/j.biopsycho.2009.09.007
Field, T., Fox, N. A., Pickens, J., & Nawrocki, T. (1995). Relative Right Frontal EEG Activation in 3- to 6-Month-Old Infants of "Depressed" Mothers. Developmental Psychology. https://doi.org/10.1037/0012-1649.31.3.358
Fox, N. A., & Davidson, R. J. (1986). Taste-elicited changes in facial signs of emotion and the asymmetry of brain electrical activity in human newborns. Neuropsychologia. https://doi.org/10.1016/0028-3932(86)90028-X
Fox, N. A., & Davidson, R. J. (1988). Patterns of Brain Electrical Activity During Facial Signs of Emotion in 10-Month-Old Infants. Developmental Psychology. https://doi.org/10.1037/0012-1649.24.2.230
Fox, N. A., Yoo, K. H., Bowman, L. C., Cannon, E. N., Ferrari, P. F., Bakermans-Kranenburg, M. J., ... Van IJzendoorn, M. H. (2016). Assessing human mirror activity With EEG mu rhythm: A meta-analysis. Psychological Bulletin. https://doi.org/10.1037/bul0000031
Gómez, A., Quintero, L., López, N., & Castro, J. (2016). An approach to emotion recognition in single-channel EEG signals: A mother child interaction. In Journal of Physics: Conference Series. https://doi.org/10.1088/1742-6596/705/1/012051
Harmon-Jones, E., & Allen, J. J. B. (1997). Behavioral activation sensitivity and resting frontal EEG asymmetry: Covariation of putative indicators related to risk for mood disorders. Journal of AbnormalPsychology. https://doi.org/10.1037/0021-843X.106.1.159
Howarth, G. Z., Fettig, N. B., Curby, T. W., & Bell, M. A. (2016). Frontal Electroencephalogram Asymmetry and Temperament Across Infancy and Early Childhood: An Exploration of Stability and Bidirectional Relations. Child Development. https://doi.org/10.1111/cdev.12466
Johnson, M. H., Dziurawiec, S., Ellis, H., & Morton, J. (1991). Newborns' preferential tracking of face-like stimuli and its subsequent decline. Cognition. https://doi.org/10.1016/0010-0277(91)90045-6
Kaneshige, T., & Haryu, E. (2015). Categorization and understanding of facial expressions in 4-month-old infants. Japanese Psychological Research. https://doi.org/10.1111/jpr.12075
Krompinger, J. W., Moser, J. S., & Simons, R. F. (2008). Modulations of the Electrophysiological Response to Pleasant Stimuli by Cognitive Reappraisal. Emotion. https://doi.org/10.1037/1528-3542.8.1.132
Leppänen, J. M., & Nelson, C. A. (2009). Tuning the developing brain to social signals of emotions. Nature Reviews Neuroscience. https://doi.org/10.1038/nrn2554
Lewis, M., Sullivan, M. W., Stanger, C., & Weiss, M. (1989). Self development and self-conscious emotions. Child Development. https://doi.org/10.1111/j.1467-8624.1989.tb02704.x
Lusby, C. M., Goodman, S. H., Bell, M. A., & Newport, D. J. (2014). Electroencephalogram patterns in infants of depressed mothers. Developmental Psychobiology. https://doi.org/10.1002/dev.21112
Mai, X., Xu, L., Li, M., Shao, J., Zhao, Z., Lamm, C., ... Lozoff, B. (2014). Sounds elicit relative left frontal alpha activity in 2-month-old infants. International Journal of Psychophysiology. https://doi.org/10.1016/j.ijpsycho.2014.09.008
Marshall, P. J., Bar-Haim, Y., & Fox, N. A. (2002). Development of the EEG from 5 months to 4 years of age. Clinical Neurophysiology. https://doi.org/10.1016/S1388-2457(02)00163-3
Marshall, P. J., & Meltzoff, A. N. (2011). Neural mirroring systems: Exploring the EEG mu rhythm in human infancy. Developmental Cognitive Neuroscience. https://doi.org/10.1016/j.dcn.2010.09.001
Maulsby, R. L. (1971). An illustration of emotionally evoked theta rhythm in infancy: Hedonic hypersynchrony. Electroencephalography and Clinical Neurophysiology. https://doi.org/10.1016/0013-4694(71)90186-6
Missana, M., & Grossmann, T. (2015). Infants' emerging sensitivity to emotional body expressions: Insights from asymmetrical frontal brain activity. Developmental Psychology. https://doi.org/10.1037/a0038469
Miyoshi, M., Katayama, J., & Morotomi, T. (2004). Face-specific N170 component is modulated by facial expressional change. NeuroReport. https://doi.org/10.1097/00001756-200404090-00035
Morgane, P. J., & Mokler, D. J. (2006). The limbic brain: Continuing resolution. Neuroscience and BiobehavioralReviews. https://doi.org/10.1016/j.neubiorev.2005.04.020
Nelson, C. A. (2001). The development and neural bases of face recognition. Infant Child Dev. https://doi.org/10.1 002/icd.239
Pascalis, O., De Haan, M., Nelson, C. A., & De Schonen, S. (1998). Long-term recognition memory for faces assessed by visual paired comparison in 3- and 6-month-old infants. Journal of Experimental Psychology: Learning Memory and Cognition. https://doi.org/10.1037/0278-7393.24.1.249
Phillips, A. T., Wellman, H. M., & Spelke, E. S. (2002). Infants' ability to connect gaze and emotional expression to intentional action. Cognition. https://doi.org/10.1016/S0010-0277(02)00073-2
Rose, S. A., Feldman, J. F., & Jankowski, J. J. (2004). Infant visual recognition memory. Developmental Review. https://doi.org/10.1016/j.dr.2003.09.004
Taylor-Colls, S., & Pasco Fearon, R. M. (2015). The Effects of Parental Behavior on Infants' Neural Processing of Emotion Expressions. Child Development. https://doi.org/10.1111/cdev.12348
van den Boomen, C., Munsters, N. M., & Kemner, C. (2017). Emotion processing in the infant brain: The importance of local information. Neuropsychologia. https://doi.org/10.1016/j.neuropsychologia.2017.09.006
Izard, C. E., & Malatesta, C. Z. (1987). Perspectives on emotional development I: Differential emotions theory of early emotional development. In J. D. Osofsky (Ed.), Wiley series on personality processes. Handbook of infant development (pp. 494-554). Oxford, England: John Wiley & Sons.
de Haan, M. (2007) Visual attention and recognition memory in infancy. In M de Haan (Ed.), Studies in developmental psychology. infant EEG and event-related potentials (pp. 101-143). New York, NY, US: Psychology Press.

## Claims

1. An apparatus, comprising: electroencephalography (***EEG***) electrodes configured to make optimum point contacts with a scalp of an infant or a young child, wherein the ***EEG*** electrodes are positioned either (1) in an arrangement of 13 electrodes at positions AF3, AF4, F5, F6, FC1, FC2, T7, T8, P7, P8, CPz, O1 and O2, or (2) in an arrangement of 17 electrodes at positions AF3, AF4, F5, F6, FC1, FC2, T7, T8, P7, P8, CP3, CP4, CPz, PO3, PO4, O1 and O2,
wherein the electrode positions are according to the nomenclature template taken from American Electroencephalographic Society guidelines, 1991; Journal of Clinical Neurophysiology, 8, pp 200-201,
further comprising a flexible support structure attached to the electrodes.

2. The apparatus of claim 1, further comprising amplifiers associated with the electrodes, wherein data from each of the electrodes is individually amplified and isolated.

3. The apparatus of claim 2, wherein the amplifiers are configured on a flexible printed circuit board.

4. The apparatus of claim 1, wherein the amplifier transmits aggregated data wirelessly to a remote data analyzer.

5. The apparatus of claim 2, wherein the amplified data is aggregated with amplified data from a plurality of other electrodes on the ***EEG headset.***

## Patentansprüche

1. Einrichtung, umfassend:
Elektroenzephalographie(EEG)-Elektroden, die dazu ausgelegt sind, optimale Punktkontakte mit einer Kopfhaut eines Säuglings oder eines Kleinkindes herzustellen, wobei die EEG-Elektroden entweder (1) in einer Anordnung von 13 Elektroden an den Positionen AF3, AF4, F5, F6, FC1, FC2, T7, T8, P7, P8, CPz, O1 und O2 oder (2) in einer Anordnung von 17 Elektroden an den Positionen AF3, AF4, F5, F6, FC1, FC2, T7, T8, P7, P8, CP3, CP4, CPz, PO3, PO4, O1 und O2 positioniert sind,
wobei die Elektrodenpositionen der Nomenklaturvorlage den Leitlinien der American Electroencephalographic Society, 1991; Journal of Clinical Neurophysiology, 8, S. 200-201, entsprechen,
ferner eine flexible Stützstruktur umfassend, die an den Elektroden angebracht ist.

2. Einrichtung nach Anspruch 1, ferner Verstärker umfassend, die mit den Elektroden assoziiert sind, wobei Daten von jeder der Elektroden einzeln verstärkt und isoliert werden.

3. Einrichtung nach Anspruch 2, wobei die Verstärker auf einer flexiblen Leiterplatte ausgelegt sind.

4. Einrichtung nach Anspruch 1, wobei der Verstärker aggregierte Daten drahtlos an einen entfernten Datenanalysator überträgt.

5. Einrichtung nach Anspruch 2, wobei die verstärkten Daten mit verstärkten Daten aus mehreren anderen Elektroden am EEG-Headset aggregiert werden.

## Revendications

1. Appareil, comprenant : des électrodes d'électroencéphalographie (EEG) configurées pour établir des contacts ponctuels optimaux avec le cuir chevelu d'un nourrisson ou d'un jeune enfant, les électrodes EEG étant positionnées (1) dans un agencement de 13 électrodes aux positions AF3, AF4, F5, F6, FC1, FC2, T7, T8, P7, P8, CPz, O1 et O2, ou (2) dans un agencement de 17 électrodes aux positions AF3, AF4, F5, F6, FC1, FC2, T7, T8, P7, P8, CP3, CP4, CPz, PO3, PO4, O1 et O2,
les positions des électrodes étant conformes au modèle de nomenclature tiré des directives de la American Electroencephalographic Society, 1991 ; Journal of Clinical Neurophysiology, 8, pp 200-201,
comprenant en outre une structure de support flexible fixée aux électrodes.

2. Appareil selon la revendication 1, comprenant en outre des amplificateurs associés aux électrodes, les données provenant de chacune des électrodes étant amplifiées et isolées individuellement.

3. Appareil selon la revendication 2, les amplificateurs étant configurés sur une carte de circuit imprimé flexible.

4. Appareil selon la revendication 1, l'amplificateur transmettant sans fil des données agrégées à un analyseur de données distant.

5. Appareil selon la revendication 2, les données amplifiées étant agrégées avec des données amplifiées provenant d'une pluralité d'autres électrodes sur le casque EEG.
